# EUROPEAN PATENT SPECIFICATION

(11) **EP 1 402 884 B1**
(45) Date of publication and mention of the grant of the patent: **31.05.2006**
(21) Application number: 03021633.7
(22) Date of filing: 25.09.2003
(51) Int. Cl.: A61K 9/127, A61K 31/4184

(54) **Liposome-containing remedy for the treatment of vascular diseases**
Liposomenhaltiges Mittel zur Behandlung Gefässerkrankungen
Medicament renfermant des liposomes pour le traitement de maladies vasculaires

(30) Priority: 25.09.2002 JP 2002278287
(43) Date of publication of application: 31.03.2004
(73) Proprietor: Fuji Photo Film Co. Ltd., Kanagawa 250-0193 (JP)
(72) Inventor: Aikawa, Kazuhiro, Minami-ashigara-shi Kanagawa 250-0193 (JP)
(74) Representative: HOFFMANN EITLE

(56) References cited:
- EP-A- 0 583 665
- EP-A- 0 742 210
- WO-A-01/82977
- WO-A-02/26716
- WO-A-95/07263
- WO-A-98/51303
- GB-A- 2 184 013
- US-A- 5 869 462

## Description

### Field of the Invention

The present invention relates to a liposome that enables accumulation of an active ingredient of a medicament for prophylactic and/or therapeutic treatment of a vascular disease caused by abnormal proliferation of vascular smooth muscle cells in a site of a vascular disease such as an vascular disease such as an arteriosclerotic lesion.

### Background Art

In recent years, patients with so-called adult diseases such as arterial sclerosis, hypertension, and diabetes mellitus have been continuously increasing with prolongation of life expectancy. In particular, patients with hyperlipidemia and arterial sclerosis derived therefrom have been remarkably increasing due to excessive intake of high calorie and high cholesterol food, which have become a serious social problem. Medications currently applied for treatment of hyperlipidemia and arterial sclerosis are those lower cholesterol in blood, and no medicament that can be expected to have potency in retracting arterial sclerosis lesions has been used clinically.

Arterial sclerosis is characterized by lesions of intimal hyperplasia and lipid accumulation in blood vessels, and it has been elucidated from recent biochemical finding that foaming of macrophages plays a main role in the formation of arterial sclerosis lesions. That is, when macrophages are foamed, vascular smooth muscle cells abnormally proliferate to cause pathological conditions of vascular intimal hyperplasia, lipid storage and the like, and thus areas of blood flow are narrowed to cause disorders. Accordingly, suppression of the foaming of macrophages may possibly prevent arterial sclerosis by inhibiting formation of arterial sclerosis lesions, or achieve radicular treatment of arterial sclerosis by retraction of arterial sclerosis lesions.

It has been suggested that an inhibitor of ACAT, an enzyme involved in intestinal absorption and metabolism of cholesterol, such as imidazole derivatives described in Bio. MEd. Chem. Lett., Vol. 5(2), 167-172 (1995) reduces blood cholesterol level and thus suppresses the foaming of macrophages in an animal experiment (for example, piperazine derivatives described in International Publication WO98/54153). However, since these compounds are directed to ACAT inhibitory activity, they do not achieve satisfactory inhibition of the foaming of macrophages, and their effects are insufficient.

Some amide compounds are reported to have ACAT inhibitory action (for example, the amide compounds disclosed in International Publication WO99/25712). However, ACAT inhibitors have recently been recognized to have various toxicities and side effects (for example, Toxycol. Pharmacol., 22, 510-518 (1994); Toxicol. Appl. Pharmacol., 140,387-392 (1996)). It is considered that medicaments for treatment of arteriosclerosis advantageously have no ACAT inhibitory action with such side effects.

EP 0 583 665 discloses certain benzimidazole derivatives capable of lowering blood cholesterol and suppressing macrophage-foaming reaction. WO 95/07263 discloses various benzimidazole derivatives for use as prostacyclin (PGI2) mimetics. The treatment of thrombosis, arteriosclerosis and hyperlipidemia is suggested. A generic reference to liposome incapsulation is made without further specification of specific liposomes. WO 98/51303 relates to a method of treating HIV or other viral infections by administering a herbicide or fungicide or derivatives thereof. In particular, benzimidazole derivatives used as herbicides or fungicides are disclosed. Generic reference to liposomes as pharmaceutical carriers is made, but no specific liposomes are disclosed. WO 02/26716 discloses benzimidazole derivatives for use in the treatment of cancer or viral infections, but does not relate to the treatment of vascular diseases. GB 2 184 013 discloses anthelminthic pharmaceutical compositions based on methyl-5-benzoyl-2-benzamidazole carbamate suitable for use in the treatment of hydatidosis and alveococcosis. US 5,869,462 relates to delivery of antisense nucleotide sequences incorporated into liposomes, in particular prior to surgical grafting of blood vessels. The document does not relate to benzimidazole derivatives. It discloses the localized application of liposomes to an area to be treated. EP 1 284 146 relates to a liposome composition containing a hydrophobic iodine compound such as 1,3,5-triiodobenzene derivative used as an X-ray contrasting medium for use in radiography. The document does not disclose any active ingredients or pharmaceutical compositions suitable for treating vascular diseases.

In view of the above, the inventors of the present invention previously developed agents for therapeutic treatment of arteriosclerosis, which suppress abnormal proliferation of vascular smooth muscle cells by suppressing forming of macrophages and thereby suppress development of arteriosclerotic lesions or degenerate arteriosclerotic lesions. Since these agents have no or almost no ACAT inhibition effect, it is expected that the aforementioned side effects can be significantly reduced (Japanese Patent Unexamined Publication (Kokai) Nos. 6-48942 and 7-228530, International Publication WO95/21160, Japanese Patent Unexamined Publication Nos. 9-31062 and 9-40669). The compounds contained in the aforementioned agents are benzimidazole compounds, and they inhibited formation of arteriosclerotic lesions or degenerated arteriosclerotic lesions in a test utilizing cholesterol-loaded systems of New Zealand white rabbits. It is considered that, if specific accumulation of these compounds in arteriosclerotic lesions is achievable, a same level of pharmacological effect can be expected even at a low dose, and thus the agents can be more valuable through reduction of side effects and the like.

### Disclosure of the Invention

An object of the present invention is to provide means that enables selective accumulation, in a site of vascular disease caused by abnormal proliferation of vascular smooth muscle cells such as arteriosclerosis and restenosis after PTCA, of a medicament for prophylactic and/or therapeutic treatment of said vascular disease. The inventors of the present invention conducted various researches to achieve the aforementioned object. As a result, they found that liposomes containing a medicament as one of membrane components, which is for prophylactic and/or therapeutic treatment of a vascular disease caused by abnormal proliferation of vascular smooth muscle cells, accumulate in vascular smooth muscle cells and foam macrophages, as major constituents of arteriosclerotic lesions. The present invention was achieved on the basis of these findings.

The present invention thus provides liposomes containing, as a membrane component, an active ingredient of a medicament for prophylactic and/or therapeutic treatment of a vascular disease caused by abnormal proliferation of vascular smooth muscle cells. According to preferred embodiments of the liposomes, there are provided the aforementioned liposomes, wherein the active ingredient is that containing a benzimidazole derivative having suppression action on foaming of macrophages; the aforementioned liposomes, which contain a lipid selected from phosphatidylcholines and phosphatidylserines as a membranes component, and the aforementioned liposomes, which contain a phosphoric acid dialkyl ester as being a diester of an alkyl containing 6 or more carbon atoms as a membrane component.

From another aspect, the present invention provides medicaments for prophylactic and/or therapeutic treatment of a vascular disease caused by abnormal proliferation of vascular smooth muscle cells, preferably medicaments for prophylactic and/or therapeutic treatment of arteriosclerosis, which comprise the aforementioned liposomes. These medicaments can be preferably administered so suppress proliferation of vascular smooth muscle cells abnormally proliferating in the presence of foam macrophages, for example, to suppress abnormal proliferation of vascular smooth muscle cells in an arteriosclerotic lesion or after PTCA.

From further aspects, the present invention provides methods for prophylactic and/or therapeutic treatment of a vascular disease caused by abnormal proliferation of vascular smooth muscle cells, which comprises the step of administering a therapeutically and/or prophylactically effective amount of the aforementioned liposomes to a mammal including human, and methods for prophylactic and/or therapeutic treatment of arteriosclerosis, which comprises the step of administering a therapeutically and/or prophylactically effective amount of the aforementioned liposomes to a mammal including human.

### BRIEF EXPLANATION OF DRAWINGS

Fig. 1 shows results of mouse vascular smooth muscle cell proliferation induced in the presence of mouse foam macrophages.
Fig. 2 shows a proliferation curve of mouse vascular smooth muscle cells obtained without addition of mouse foam macrophages.
Fig. 3 shows proliferation curves of vascular smooth muscle cells obtained with addition of liposomes containing Compound (25) as a membrane component or a solution of Compound (25) in DMSO.
Fig. 4 shows inhibitory effect of the liposomes of the present invention (containing Compound (25) as a membrane component) against arteriosclerotic lesion formation in a rat arteriosclerosis pathological model.

### Best Mode for Carrying out the Invention

The liposome of the present invention is characterized to contain, as a membrane component, an active ingredient of a medicament for prophylactic and/or therapeutic treatment of a vascular disease caused by abnormal proliferation of vascular smooth muscle cells. The type of the active ingredient of a medicament for prophylactic and/or therapeutic treatment of a vascular disease caused by ahnormal proliferation of vascular smooth muscle cells is not particularly limited. For example, the active ingredient may preferably those can suppress foaming of macrophages. Bebzimidazole derivatives that strongly suppress foaming of macrophages and have reduced side effect are known, and they can be most preferably used for manufacture of the liposome of the present invention (Japanesse Patent Unexamined Publication Nos. 6-48942 and 7-228530, International Patent Publication WO95/21160, Japanese Patent Unexamined Publication, Nos. 9-31062 and 9-40669).

Those skilled in art can readily determine, by the methods described in the aforementioned patent documents, whether or not a substance has an inhibitory action on proliferation of vascular smoothy muscle cells, preferably whethes or not a certain substance inhibits foaming of macrophages. Therefore, any substance that is determined to have an inhibitory action on proliferation of vascular smooth muscle cells by those methods can be used for the manufacture of the liposome of the present invention as an active ingredient of a medicament for prophylactic and/or therapeutic treatment of a vascular disease caused by abnormal proliferation of vascular smooth muscle cells. A substance in a free form may be used as the aforementioned active ingredient of the medicament, or a substance in the form of a salt may be used.

The term "liposome containing an active ingredient of a medicament as a membrane component" referred to an the specification means that the active ingredient of a medicament is localized in a membrane that constitute the liposome. However, a mode of the active ingredient localized in the membrane constituting the liposome is not particularly limited. For example, the active ingredient may be embedded in a lipid bilayer, or may exist in an aqueous phase existing between the lipid bilayers. Further, the term "liposome containing an active ingredient of a medicament as a membrane component" is intended not to exclude that the active ingredient is contained as an aqueous solution in an aqueous phase inside the liposome, and a liposome which contains an active ingredient of a medicament as a membrane component and also contains the active ingredient of a medicament as an aqueous solution in an aqueous phase inside the liposome also fall within the scope of the present invention. An amount of the aforementioned active ingredient is not particularly limited. The amount may generally be about from 10 to 90 mass %, preferably from 10 to 80 mass %, further preferably from 20 to 80 mass %, on the basis of the total mass of membrane components of the liposome (numerical ranges defined with "from and to" in the specification are those including values of lower and upper limits).

Types of the membrane components of the liposome are not particularly limited, and any of lipid compounds ordinarily used in this field can be used. For example, those described in Biochim. Biophys. Acta, 150 (4), 44 (1982); Adv. in Lipid. Res., 16(1)1(1978); "RESEARCH IN LIPOSOMES", P. Machy, L. Leserman, John Libbey EUROTEXT Co.); "Liposome" (Ed., Nojima, Sunamoto and Inoue, Nankodo) and the like can be used. As the membrane components, phospholipids are preferred, and phosphatidylcholines (PC) are particularly preferred. Preferred examples of phosphatidylcholines include egg PC, dimyristoyl-PC (DMPC), dipalmitoyl-PC (DPPC), distearoyl-PC (DSPC), dioleyl-PC (DOPC) and the like. However, PCs are not limited to these examples.

According to another preferred embodiment of the liposome of the present invention, PC and a phosphatidylserine (PS) can be used in combination as membrane components of the liposome. For that purpose, a molar ratio of PC and PS (PC:PS) used may preferably be in a range of 90:10 to 10:90, further preferably 30:70 to 70:30.

According to a further preferred embodiment of the liposome of the present invention, a phosphoric acid dialkyl ester may be added to the combination of PC and PS as a membrane component. The two alkyl groups constituting the dialkyl ester of phosphoric acid may be the same or different, and they are preferably of the same alkyls. Numbers of carbon atoms in the alkyl group are not particularly limited. The number may be, for example, 6 or more, preferably 10 or more, and more preferably 12 or more. Preferred examples of the phosphoric acid dialkyl ester include, but not limited thereto, dilauryl phosphate, dimyristyl phosphate, dicetyl phosphate and the like. A preferred amount of the phosphoric acid diakyl ester may be from 1 to 50 mass % preferably from 1 to 30 mass %, further preferably from 1 to 20 mass %, on the basis of the total mass of PC and PS.

When liposomes containing 4 kinds of substances as membrane components, i.e., PC, PS, the phosphoric acid dialkyl ester and the active ingredient of a medicament for prophylactic and/or therapeutic treatment of a vascular disease caused by abnormal proliferation of vascular smooth muscle cells, are prepared, a preferred ratio can be chosen from 5 to 40 mass % from 5 to 40 mass %, from 1 to 10 mass % and from 15 to 80 mass %, respectively.

As the components of the liposome of the present invention, any components other than those explained above can be used. Examples of such components include cholesterol, cholesterol esters, sphingomyelin, monosial ganglioside GM1 derivatives described in FEBS Lett., 223, 42 (1987); Proc. Natl. Acad. Sci. USA, 85, 6949 (1988) etc., glucuronic acid derivatives described in Chem. Lett., 2145 (1988); Biochim. Biophys. Acta, 1148, 77 (1992) and the like, polyethylene glycol derivatives described in Biochim. Biophys Acta, 1029, 91 (1990); FEBS Lett. 268, 235 (1980) and the like. However, the components are not limited to these examples.

The liposome of the present invention can be prepared by any methods available in this field. The preparation methods describes in Ann. Rev. Biophys. Bioeng., 9, 467 (1980),"Liopsomes" (Ed. by M.J. Ontro, MARCELL DEKKER, INC.) and the like, as well as the published reviews of liposomes mentioned above can be used. More specifically, examples include the ultrasonication method, ethanol injection method, French press method, ether injection method, cholic acid method, calcium fusion method, freeze and thawing method, reverse phase evaporation method and the like. However, the preparation methods are not limited to these examples.

Size of the liposome may be any of those obtainable by the aforementioned methods. Generally, a size in average may be 400 nm or less, preferably 200 nm or less. Structure of the liposome is not also particularly limited, and may be any structure such as unilamellar or multilamellar structure. As a solution contained in the liposome, buffer, physiological saline and the like can be used as well as water. The aforementioned solutions may be used with addition of an appropriate amount of a water-soluble organic solvent (e.g., glycerin).

The medicaments containing the liposomes of the present invention as an active ingredient can be used as medicaments for prophylactic and/or therapeutic treatment of a vascular disease caused by abnormal proliferation of vascular smooth muscle cells, and can be orally or parenterally administered to a mammal including human. Examples of parenteral administration include intrabronchial, intrarectal, subcutaneous, intramuscular, intravenous administrations ad the like. Examples of pharmaceutical preparation suitable for oral administration include, for example, tablets, granules, subtilized granules, powders, syrups, solutions, capsules, chewable tablets, suspensions and the like, and examples of pharmaceutical preparations suitable for parenteral administration include, for example, injections, fusion drips, inhalants, sprays, suppositories, transdermal preparations, transmucosal preparations, eye drops, ear drops, nose drops, patches and the like. It is also possible to provide liquid preparations such as injections and fusion drips as, for example, lyophilized powdery compositions, and suspend the compositions in water or other suitable medium (for example, physiological saline, glucose infusion, buffer and the like) upon use.

Doses and administration frequencies of the medicaments of the present invention are not particularly limited, and suitable doses and administration frequencies can be determined depending on various factors such as type of disease, age, body weight and symptoms of patients and the like. As for oral administration, the medicament can be administered once to several times a day for an adult in an amount of 0.1 to 1000 mg/kg as a weight of the active ingredient per day. As for parenteral administration, the medicament may preferably be administered once to several times a day for an adult in an anount of 0.001 to 100 mg/kg as a weight of the active ingredient per day.

It is kwown that, in vascular diseases such as arteriosclerosis or restenosis after PTCA, vascular smooth muscle cells abnormally proliferate and migrate into endosporium at the same time to narrow blood flow passages. Factors triggering the abnormal proliferation of normal vascular smooth muscle cells have not yet been clearly elucidated, however, it is known that migration of macrophages into endosporium and foaming thereof are important factors. It is reported that vascular smooth muscle cells then cause phenotype conversion (from constricted to composite type). As specifically demonstrated in the following examples, when the liposomes of the present invention are used, the aforementioned active ingredient of a medicament can be selectively taken up into the vascular smooth muscle cells that begin to abnormally proliferate in the presence of foam macrophagee. As a result, the abnormal proliferation of vascular smooth muscle cells can be inhibited, thereby development of arteriosclerotic lesions can be suppressed, and/or arteriosclerotic lesions can be degenerated.

### Examples

The present invention will be explained more specifically with reference to the following examples. However, the scope of the present invention is not limited to the following examples.

### Example 1: Preparation of culture system of vascular smooth muscle cells of which proliferation is activated by foam macrophages

Vascular smooth muscle cells were isolated from mouse aorta endothelium ("Tissue Culture Method", 10th Edition, ed. by the Japenese Tissue Culture Association, published by Kodansha, 1998). The isolated vascular smooth muscle cells were suspended in 10% FBS Eagle's MEM (GIBCO, No. 11095·080) and inoculated in wells of a 12-well microplate(FALCON, No. 3508). The number of the cells in each well was adjusted to 10,000 cells. The cells were cultured for 3 days under conditions of 37°C and 5% CO₂.

Then, foamed mouse peritoneal macrophages were prepared according to the method described in Biochimica Biophysica Acta, 1213, 127-134 (1994). 200,000 cells of the foam macrophages were separated and inoculated on an insert cell (FALCON, No. 3180) placed on each well of the microplate where the vascular smooth muscle cells were cultured on the bottom surface. The cells were cultured for 5 days under conditions of 37°C and 5% CO₂.

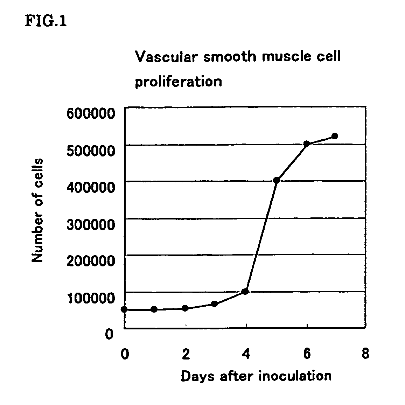

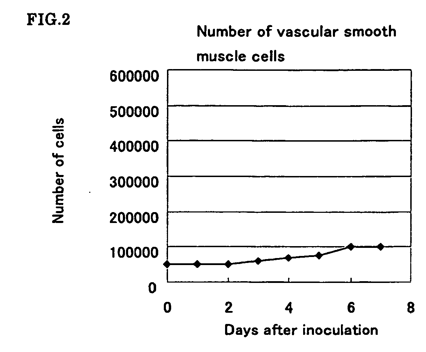

The cell numbers of the vascular smooth muscle cells in the above experiment are shown in Fig. 1. Although the vascular smooth muscle cells gently proliferated at an early stage after the start of the culture, they actively proliferated after the addition of the foam macrophages on the 3rd day and a subsequent induction period of about 1 day. A proliferation curve of vascular smooth muscle cells not added with the macrophages is shown in Fig. 2. Comparison of the results shown in Figs. 1 and 2 clearly indicates activating effect of the foam macrophages on the proliferation.

### Example 2: Preparation of liposomes

A mixture of egg PC (50 nmol, Funakoshi, No. 1201-41-0214), egg PS (50 nmol, Funakoshi, No.1201-42-0226) and a compound synthesized by the method described in Japanese Patent Application No. 11-260384 (Compound 25, 20 nmol) was dissolved in chloroform contained in an eggplant-shaped flask to form a uniform solution, and then the solvent was evaporated under reduced pressure to form a thin membrane on the bottom of the flask. The thin membrane was dried in vacuo, then added with 1.5 ml of 0.9% physiological saline (Hikari Pharmaceutical, No. 512) and ultrasonicated (probe type oscillator, Branson No. 3542, 0.1 mW) for 5 minute with ice cooling to obtain a uniform liposome dispersion (referred to as "Preparation 1" hereinafter). Size of the particles contained in the obtained dispersion was measured by using WBC analyzer (Nihon Kohden, A-1042). The particle size was from 40 to 65 nm.

### Example 3: Suppression of proliferation of vascular smooth muscle cells

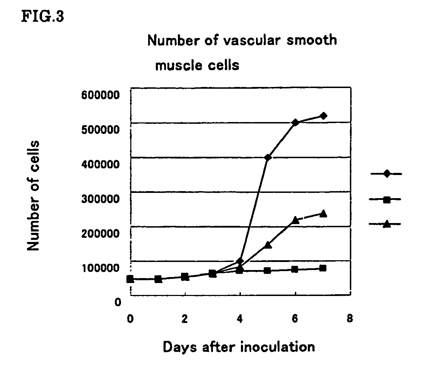

Compound 25 was added at a concentration of 1 µM in the form of Preparation 1 prepared in Example 2 or a solution in DMSO (Wako Pure Chemical Industries, No. 043-07216) to the medium of the vascular smooth muscular cell culture system prepared in Example 1 in which cell proliferation was activated by foam macrophages 24 hours after the foam macroghages were inoculated into the insert cell. The same system except that only the same amount of DMSO was added was used as a control. The numbers of the vascular smooth muscle cells of the control were taken as 100, and changes with passage of time of numbers of the vascular smooth muscle cells of the system added with Preparation 1 and those of the system added with DMSO solution are shown in Fig. 3. In the system added with Preparation 1, the proliferation of vascular smooth muscle cells was more remarkably suppressed compared with that of the system added with DMSO solution. In this experiment, it was also verified from morphological observation of the cells that no toxicity was expressed in the macrophages and the smooth smooth muscle cells.

### Example 4: Inhibitory effect on formation of arteriosclerotic lesion in rat arteriosclerosis pathological model

Nine SD male rats (6-week old, Charles River japan) were acclimatized for 1 week. The rates were subjected to partial thoracotomy under anesthesia with nembutal (RAVONAL, Tanabe Pharmaceutical), and a balloon catheter (Natsume Kagaku, No. 21-2045) was inserted to form a wound of about 1 cm on the aortic arch. After the formation of the wound, the following tests were simultaneously performed (n = 3 for each test).
Test. 1: The rats were fed with 0.5% cholesterol-loaded feed (Oriental Yeast) for 14 days.
Test 2: The rats were fed with 0.5% cholesterol-loaded feed for 14 days. At the same time, Preparation 1 was continuously administered (Compound 25, 1 mg/kg/day) for 14 days (injected into caudal vein).
Test 3: The rats were fed with 0.5% cholesterol-loaded feed for 14 days. At the same time, Compound 25 dissolved in 10% DMSO and 90% physiological saline (Hikari Pharmaceutical) was continuously administered at a dose of 1 mg/kg/day for 14 days (injected into caudal vein).

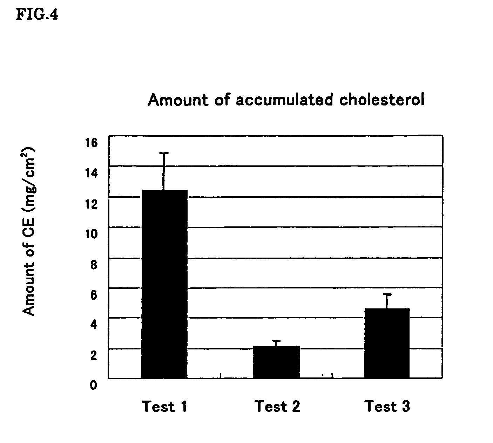

After completion of the tests, the aortic arches were extracted, and cholesterol esters accumulated on the vascular inner walls were quantified (Fig. 4). It is clear that the amount of accumulated cholesterol esters in Test 2 was significantly decreased compared with that in Test 3.

The liposomes of the present invention can allow an active ingredient of a medicament, which is for prophylactic and/or therapeutic treatment of a vascular disease caused by abnormal proliferation of vascular smooth muscle cells, accumulated in a site of a vascular disease such as an arteriosclerotic lesion. The medicaments containing the liposomes of the present invention can exhibit superior effect in prophylactic and/or therapeutic treatment of a vascular disease caused by abnormal proliferation of vascular smooth muscle cells.

## Claims

1. A liposome containing as a membrane component an active ingredient for prophylactic and/or therapeutic treatment of a vascular disease caused by abnormal proliferation of vascular smooth muscle cells, wherein the active ingredient comprises a benzimidazole derivative having suppressing action on foaming of macrophages, and wherein said liposome contains as a membrane component a lipid selected from phosphatidylcholines and phosphatidylserines.

2. The liposome according to claim 1, which contains as a membrane component a phosphoric acid dialkyl ester as being a diester of an alkyl containing 6 or more carbon atoms.

3. The liposome according to claim 1 or 2, wherein said vascular disease is arteriosclerosis.

4. Use of a liposome which contains as a membrane component a benzimidazole derivative having suppressing action on foaming of macrophages as an active ingredient, and which contains as a membrane component a lipid selected from phosphatidylcholines and phosphatidylserines, for the preparation of a medicament for the prophylactic and/or therapeutic treatment of a vascular disease caused by abnormal proliferation of vascular smooth muscle cells.

5. The use according to claim 4, wherein the liposome contains as a membrane component a phosphoric acid dialkyl ester as being a diester of an alkyl containing 6 or more carbon atoms.

6. The use according to claim 4 or 5, wherein said vascular disease is arteriosclerosis.

## Patentansprüche

1. Liposom, das als Membrankomponente einen Wirkstoff für die prophylaktische und/oder therapeutische Behandlung einer vaskulären Erkrankung enthält, die durch abnormale Proliferation vaskulärer glatter Muskelzellen ausgelöst wird, wobei der Wirkstoff ein Benzimidazolderivat mit einer supprimierenden Wirkung auf die Bildung aktivierter Makrophagen umfasst und wobei das Liposom als Membrankomponente ein Lipid enthält, gewählt aus Phosphatidylcholinen und Phosphatidylserinen.

2. Liposom gemäss Anspruch 1, das als Membrankomponente einen Phosphorsäuredialkylester enthält, als Diester eines Alkyls, enthaltend 6 oder mehr Kohlenstoffatome.

3. Liposom gemäss Anspruch 1 oder 2, wobei die vaskuläre Erkrankung Arteriosklerose ist.

4. Verwendung eines Liposoms, das als Membrankomponente ein Benzimidazolderivat mit supprimierender Wirkung auf die Bildung aktivierter Makrophagen als Wirkstoff enthält, und das als Membrankomponente ein Lipid enthält, gewählt aus Phosphatidylcholinen und Phosphatidylserinen, für die Herstellung eines Medikaments für die prophylaktische und/oder therapeutische Behandlung einer vaskulären Erkrankung, die durch eine abnormale Proliferation vaskulärer glatter Muskelzellen ausgelöst wird.

5. Verwendung gemäss Anspruch 4, wobei das Liposom als Membrankomponente einen Phosphorsäuredialkylester als Diester eines Alkyls, enthaltend 6 oder mehr Kohlenstoffatome, enthält.

6. Verwendung gemäss Anspruch 4 oder 5, wobei die vaskuläre Erkrankung Arteriosklerose ist.

## Revendications

1. Liposome contenant en tant que composant membranaire un ingrédient actif pour un traitement prophylactique et/ou thérapeutique d'une maladie vasculaire provoquée par une prolifération anormale des cellules musculaires lisses vasculaires, dans lequel l'ingrédient actif comprend un dérivé benzimidazole ayant une action de suppression sur la transformation des macrophages en cellules spumeuses, et dans lequel ledit liposome contient en tant que composant membranaire un lipide choisi parmi des phosphatidylcholines et des phosphatidylsérines.

2. Liposome selon la revendication 1, qui contient en tant que composant membranaire un ester de dialkyle d'acide phosphorique qui est un diester d'un alkyle contenant 6 atomes de carbone ou plus.

3. Liposome selon la revendication 1 ou 2, dans lequel ladite maladie vasculaire est l'artériosclérose.

4. Utilisation d'un liposome qui contient en tant que composant membranaire un dérivé benzimidazole ayant une action de suppression sur la transformation des macrophages en cellules spumeuses en tant qu' ingrédient actif, et qui contient en tant que composant membranaire un lipide choisi parmi des phosphatidylcholines et des phosphatidylsérines, pour la préparation d'un médicament pour le traitement prophylactique et/ou thérapeutique d'une maladie vasculaire provoquée par une prolifération anormale des cellules musculaires lisses vasculaires.

5. Utilisation selon la revendication 4, dans laquelle le liposome contient en tant que composant membranaire un ester de dialkyle d'acide phosphorique qui est un diester d'un alkyle contenant 6 atomes de carbone ou plus.

6. Utilisation selon la revendication 4 ou 5, dans laquelle ladite maladie vasculaire est l'artériosclérose.
